# EUROPEAN PATENT SPECIFICATION

(11) **EP 4 230 200 B1**
(45) Date of publication and mention of the grant of the patent: **11.06.2025**
(21) Application number: 22767148.4
(22) Date of filing: 08.03.2022
(51) Int. Cl.: A61K 31/198, A61P 13/12, A61K 9/00

(54) **AGENT FOR INHIBITING RENAL DAMAGE INDUCED BY RHABDOMYOLYSIS**
MITTEL ZUR HEMMUNG VON DURCH RHABDOMYOLYSE INDUZIERTER NIERENSCHÄDIGUNG
AGENT POUR INHIBER LES LÉSIONS RÉNALES INDUITES PAR LA RHABDOMYOLYSE

(30) Priority: 12.03.2021 JP 2021040102
(43) Date of publication of application: 23.08.2023
(73) Proprietor: Niigata University, Niigata 950-2181 (JP); Denka Company Limited, Tokyo 103-8338 (JP)
(72) Inventor: SAITO, Akihiko, Niigata-shi, Niigata 951-8510 (JP); GOTO, Sawako, Niigata-shi, Niigata 951-8510 (JP); HIRAYAMA, Yoshiaki, Tokyo 103-8338 (JP)
(74) Representative: Vossius & Partner Patentanwälte Rechtsanwälte mbB
(86) International application number: PCT/JP2022/010037
(87) International publication number: WO 2022/191194

(56) References cited:
- WO-A1-2015/111666
- JP-A- 2017 535 522
- JP-A- 2018 524 402
- US-A1- 2019 262 321
- K. MATSUSHITA: "Megalin deletion prevents long-term loss of glomerular filtration rate after rhabdomyolyisis", 26 October 2018 (2018-10-26), XP093158616, Retrieved from the Internet <URL:https://www.asn-online.org/education/kidneyweek/2018/program-abstract.aspx?controlId=3019457>
- YOSHIHISA HORI ET AL: "Megalin Blockade with Cilastatin Suppresses Drug-Induced Nephrotoxicity", JOURNAL OF THE AMERICAN SOCIETY OF NEPHROLOGY, WILLIAMS AND WILKINS, BALTIMORE, MD, US, vol. 28, no. 6, 1 June 2017 (2017-06-01), pages 1783 - 1791, XP009194737, ISSN: 1046-6673, DOI: YOSHIHISA HORI, NOBUMASA AOKI,SHOJI KUWAHARA
- OLIVIER BOUTAUD: "Acetaminophen inhibits hemoprotein-catalyzed lipid peroxidation and attenuates rhabdomyolysis-induced renal failure", PROCEEDINGS OF THE NATIONAL ACADEMY OF SCIENCES, vol. 107, no. 6, 1 February 2010 (2010-02-01), pages 2699 - 2704, XP093158564, ISSN: 0027-8424, DOI: 10.1073/pnas.0910174107
- ZOROVA LJUBAVA D ET AL: "The role of myoglobin degradation in nephrotoxicity after rhabdomyolysis", CHEMICO-BIOLOGICAL INTERACTIONS, ELSEVIER SCIENCE IRLAND, IR, vol. 256, 18 June 2016 (2016-06-18), pages 64 - 70, XP029675576, ISSN: 0009-2797, DOI: 10.1016/J.CBI.2016.06.020
- JAKUB GBUREK, HENRIK BIRN, PIERRE J. VERROUST, BOGUSLAWA GOJ, CHRISTIAN JACOBSEN, SØREN K. MOESTRUP, THOMAS E. WILLNOW, AND ERIK I: "Renal uptake of myoglobin is mediated by the endocytic receptors megalin and cubilin.", AJP: RENAL PHYSIOLOGY, AMERICAN PHYSIOLOGICAL SOCIETY, US, vol. 285, no. 3, 1 September 2003 (2003-09-01), US , pages F451 - F458, XP008150897, ISSN: 0363-6127, DOI: 10.1152/ajprenal.00062.2003

## Description

### TECHNICAL FIELD

The present invention relates to inhibition of renal injuries induced by rhabdomyolysis. More specifically, this invention relates to an inhibitor for renal injuries induced by rhabdomyolysis, the inhibitor comprising cilastatin or a pharmaceutically acceptable salt thereof. The present invention also relates to an inhibitor for myoglobin uptake into kidney tissues.

### BACKGROUND ART

It is known that when a large amount of free myoglobin is released into blood by rhabdomyolysis, the myoglobin is transported to kidneys and causes injuries in renal tissues, thereby leading to renal injuries.

One of the mechanisms of renal injuries currently presumed is that myoglobin taken up into renal tubular epithelial cells produces reactive oxygen species through the iron contained in heme and it causes cell injuries. Further, heme freed from myoglobin is known to be released in blood and urine, and it is thus believed that free heme also causes injuries in renal proximal tubule.

It is said that the renal proximal tubular injuries caused by myoglobin, heme, and iron derived from rhabdomyolysis trigger the renal tubular obstruction due to columnar shape cell components dropped out by the injuries thereby leading to the onset of acute renal failure.

The renal injuries induced by myoglobin and heme derived from rhabdomyolysis cause the onset of acute kidney injury (AKI). AKI, in most cases, is transient and reversible but can be aggravated in some cases and seriously affects prognosis, and further transition from AKI to chronic kidney disease is known in some cases. Further, in the case developing AKI because of a medical procedure of some kind, AKI can possibly disturb the original therapy.

Thus, means for inhibiting renal injuries induced by rhabdomyolysis is in demand.

It is reported that **α**₁-microglobulin (**α**₁M) and hemopexin (Hx), which are proteins produced in the liver, bind to free heme and prevent reactive oxygen production (NPL 1). Additionally, it is also reported the use of recombinant hemopexin for the purpose of reducing the heme toxicity (PTL 1). However, according to NPL 1, the heme-**α**₁M complex possibly causes renal injuries when excessively taken up into kidney tissues.

It is reported that cilastatin can inhibit renal injuries caused by some drugs by antagonizing the megalin receptor against the drugs binding to megalin, which is expressed in renal proximal tubular cells (NPL 2, PTL 2).

K. Matsushita (2018-10-26 URL:https://www.asn-online.org/education/kidneyweek/2018/program-abstract.aspx?controlId=3019457) discloses that proximal tubule-specific deletion of megalin ameliorates chronic renal functional loss caused by experimental rhabdomyolysis.

WO 2015/111666 A1 discloses an agent for preventing a kidney disorder or inner ear disorder induced through megalin by a megalin ligand, the agent comprising cilastatin as an active ingredient.

### CITATION LIST

### PATENT LITERATURE

PTL 1: Japanese Patent Translation of PCT International Application Publication No. 2017-531643
PTL 2: International Patent Publication No. WO2019/208777

### NON PATENT LITERATURE

NPL 1: Zagar et al., 2016, American Journal of Physiology-Renal Physiology, vol. 311, p.640-651
NPL 2: Hori et al., Journal of the American Society of Nephrology, 2017, vol. 28, p.1783-1791

### SUMMARY OF INVENTION

### TECHNICAL PROBLEM

In one aspect of the present invention, an object of the present invention is to provide a novel means for inhibiting renal injuries induced by rhabdomyolysis.

In another aspect of the present invention, an object of the present invention is to provide a novel means for inhibiting myoglobin uptake into kidney tissues.

### SOLUTION TO PROBLEM

The present inventors have conducted intensive studies to achieve the aforementioned objects and as a result found that cilastatin ((Z)-7-[[(R)-2-Amino-2-carboxyethyl]thio]-2-[[[(S)-2,2-dimethylcyclopropyl]carbonyl]amino]-2-heptenoic acid) is effective to accomplish the aforementioned objects.

The present invention includes, but is not limited to, the following embodiments.
1. An inhibitor for renal injuries induced by rhabdomyolysis, comprising cilastatin or a pharmaceutically acceptable salt thereof as an active component.
2. The inhibitor according to 1, wherein the inhibitor is in an injectable form.
3. An inhibitor for myoglobin uptake into kidney tissues, comprising cilastatin or a pharmaceutically acceptable salt thereof as an active component.

### ADVANTAGEOUS EFFECTS OF INVENTION

The present invention can inhibit renal injuries induced by rhabdomyolysis.

It is considered that myoglobin freed by rhabdomyolysis is taken up into kidney tissues through the mediation of megalin and causes renal injuries. The present inventors confirmed that cilastatin inhibits the binding between megalin and myoglobin and inhibits the myoglobin uptake into kidney tissues. It is considered that the present invention inhibits renal injuries through this mechanism.

Additionally, the present inventors confirmed that α₁M and Hx to which heme freed from myoglobin binds bind to megalin, and cilastatin inhibits the binding between α₁M or Hx and megalin. Thus, heme is intracellularly absorbed through the mediation of megalin and causes renal injuries, but it is confirmed that cilastatin inhibits the binding between α₁M or Hx and megalin. For this reason, it is considered that cilastatin can inhibit heme uptake into kidney tissues. It is then considered that the present invention can not only inhibit the injuries caused by myoglobin freed by rhabdomyolysis but also inhibit injuries caused by heme.

The present invention is also advantageous in the aspects of safety and price. First of all, a combination agent of cilastatin and an antibiotic imipenem has been safely used for many years across the world. This verifies the safety of cilastatin. The drug price of the above combination agent (including the main agent) in Japan (in terms of the standard single dose based on the package insert) is 1,741 JP Yen.

As used herein in connection with renal injuries, the term "inhibit (inhibiting or inhibition)" refers to, for example, complete prevention of the onset of a symptom, or reduction of a symptom. The term "reduction" as referred to above includes decreasing the degree of the onset of the symptom, decreasing the symptom occurred, and complete elimination of the symptom occurred. Herein, complete prevention of the onset or reduction of the degree of the onset of a disease symptom is referred to as "prevention". Further, a medicament for "inhibiting" may also be referred to as an "inhibitor".

These acknowledgements in respect of the "inhibition" also apply *mutatis mutandis* to the "inhibition" of uptake.

### BRIEF DESCRIPTION OF DRAWINGS

Fig. 1 shows the renal injury inhibitory effect of cilastatin in rhabdomyolysis model mice in terms of measured values of blood potassium.
Fig. 2-1 shows, using the quartz crystal microbalance (QCM) method, the binding property of megalin onto the myoglobin- or Hx-immobilized quartz crystal sensor, and the inhibitory effect of cilastatin.
Fig. 2-2 shows, using the QCM method, the binding property of α₁M onto the megalin-immobilized quartz crystal sensor, and the inhibitory effect of cilastatin.
Fig. 3 shows the comparison of urinary protein (α₁M) concentrations between the cilastatin-treated group and control group.
Fig. 4 shows the images comparing the amounts of heme-derived iron taken up into kidney tissues between rhabdomyolysis models using kidney-specific megalin KO mice and control mice (Berlin blue tissue staining).
Fig. 5 shows the myoglobin uptake inhibitory effect by cilastatin in rhabdomyolysis model mice (immunostaining by anti-myoglobin antibody).
Fig. 6 shows the degrees of injuries in kidney tissues in rhabdomyolysis model mice using kidney-specific megalin KO mice and control mice (immunostaining by an anti-Kidney Injury Molecule-1 (KIM-1 antibody)).

### DESCRIPTION OF EMBODIMENTS

### (Renal injuries induced by rhabdomyolysis)

In one aspect, the present invention is directed to an inhibitor for renal injuries induced by rhabdomyolysis.

Rhabdomyolysis refers to the breakdown or necrosis of skeletal muscle cells caused by a traumatic or non-traumatic cause. Muscle cell components such as myoglobin released into blood during the breakdown trigger acute renal failure and various complications.

Examples of causes of rhabdomyolysis include traumatic causes such as intense exercise, heat stroke, epileptic seizure, contusion syndrome, burn, electric shock, extended cardiopulmonary resuscitation, repetitive defibrillation, embolization, anterior tibial muscle syndrome, and sickle cell disease; and non-traumatic causes such as drug myopathies caused by lipid-lowering drugs, antibiotics, sedative drugs, or the like.

When there is the above cause in the background, along with a subjective symptom such as loss in muscle strength, fatigue, or sore muscle, rhabdomyolysis may have been occurred.

The inhibitor of the present invention can be prescribed when rhabdomyolysis is diagnosed to have occurred, or suspected to have occurred. Rhabdomyolysis can be diagnosed or suspected to have occurred when, for example, clinical examinations have findings of hypermyoglobinemia, hyperkalemia, an elevated creatine kinase (CK) level in blood or abnormal coagulation, or have a distinctive urinary finding of dark brown urine due to myoglobin.

Further, in the case where the onset of renal injuries by rhabdomyolysis is concerned, particularly in subjects with a reduced renal function, the inhibitor can be prescribed as prevention.

Additionally, examples of inhibition of renal injuries induced by rhabdomyolysis concerned at clinical sites include, in terms of clinical examinations, inhibition of an elevated serum creatinine level, a reduced renal filtration function (GFR, eGFR), an elevated blood urea nitrogen level, an elevated blood uric acid level, or ion concentration abnormality in blood or urea, and also inhibition of urinary concentration abnormality of, so-called renal injury markers, KIM-1, N-acetyl-β-D-glucosaminidase (NAG), α₁M, β₂-microglobulin (β2M), Liver-type Fatty Acid-Binding Protein (L-FABP), Neutrophil Gelatinase-Associated Lipocalin (N-GAL), albumin (ALB), type IV collagen, megalin, or podocalyxin.

Additionally, examples of inhibition of renal injuries from a viewpoint of clinical symptoms include inhibition of edema, hypertension, uremia symptoms (nausea and vomiting, consciousness disturbance and the like), or jaundice, inhibition of appearance of hematuria, hemoglobinuria, or proteinuria, or inhibition of reduction in urine volume, and examples of inhibition of renal injuries from a pathological viewpoint include inhibition of renal tubular epithelial injuries, cast formation, renal tubular obstruction, stromal cell infiltration/edema/fibrosis, glomerular injury, or angiopathy.

These renal injuries can be induced through the mediation of megalin. Since the main site of expression of megalin in the body is renal proximal tubular epithelial cells (mainly luminal plasmalemma), the present invention is useful for the inhibition (i.e., as an inhibitor) of renal proximal tubular epithelial cell injury and renal injuries derived therefrom.

Examples of renal injuries induced by rhabdomyolysis include nephropathy, renal injury, nephritis, renal failure, renal disease, acute nephropathy, acute kidney injury, acute nephritis, acute renal failure, acute renal disease, chronic nephropathy, chronic renal injury, chronic nephritis, chronic renal failure, chronic kidney disease, tubular nephropathy, tubular renal injury, tubular nephritis, tubular renal failure, tubular renal disease, tubulointerstitial nephropathy, tubulointerstitial renal injury, tubulointerstitial nephritis, tubulointerstitial renal failure, tubulointerstitial renal disease, obstructive nephropathy, obstructive renal injury, obstructive nephritis, obstructive renal failure, obstructive renal disease, acute nephritic syndrome, rapidly progressive nephritic syndrome, chronic nephritic syndrome, nephrotic syndrome, renal vasospasm, and acute tubular necrosis.

### (Cilastatin)

In the present invention, cilastatin or a pharmaceutically acceptable salt thereof is used.

Cilastatin refers to (Z)-7-[[(R)-2-Amino-2-carboxyethyl]thio]-2-[[[(S)-2,2-dimethylcyclopropyl]carbonyl]amino]-2-heptenoic acid. For the sake of confirmation, when cilastatin produces a hydrate, use of the hydrate is also included within the scope of this invention.

Examples of a pharmaceutically acceptable salt of cilastatin include alkali metal salts, such as lithium salt, sodium salt and potassium salt; alkali earth metal salts, such as magnesium salt and calcium salt; zinc salt and aluminum salt; organic amine salts, such as choline salt, ethanolamine salt, trimethylamine salt, triethylamine salt, dicyclohexylamine salt, dibenzylamine salt, phenethylbenzylamine salt, procaine salt, morpholine salt, pyridine salt, piperidine salt, piperadine salt and N-ethylpiperidine salt; ammonium salt; basic amino acid salts, such as lysine salt and arginine salt. A particularly preferred salt is cilastatin sodium. For the sake of confirmation, the scope of pharmaceutically acceptable salts also includes hydrates of the salts.

As cilastatin or a pharmaceutically acceptable salt thereof, use can be made of, for example, a commercially available product, or a product produced or obtained by a known method or by a method pursuant to a known method.

Cilastatin binds to the extracellular region of megalin. Cilastatin or a pharmaceutically acceptable salt thereof can inhibit the direct or indirect binding of myoglobin or heme to megalin, and the uptake of them into cells.

The inhibitor of the present invention contains cilastatin or a pharmaceutically acceptable salt thereof in an effective amount for inhibiting renal injuries induced by rhabdomyolysis. In the case of renal injuries, an exemplary daily dose of cilastatin or a salt thereof in an adult is from 0.01 to 100 mg, or from 0.1 to 10 mg. To achieve a dose in such a range, the inventive inhibitor can be administered once or in divided doses. The inhibitor may also be administered using an intermittent dosing, such as alternate-day or every three day dosing.

### (Inhibitor for myoglobin uptake into kidney tissues)

The present invention, in an embodiment, relates to an inhibitor for myoglobin uptake into kidney tissues, comprising cilastatin or a pharmaceutically acceptable salt thereof as an active component.

Myoglobin is a protein present in muscles of vertebrates including human and includes a single polypeptide moiety called globin and a single heme moiety, which is a prosthetic group. Myoglobin has the nature of binding to oxygen molecules, and plays a role of supplying oxygen to the muscles.

**In** connection with the present invention, the present inventors found that cilastatin inhibits the binding between myoglobin and megalin. It is considered that this inhibitory effect leads to inhibition of renal injuries induced by rhabdomyolysis.

The inhibitor of the present invention contains cilastatin or a pharmaceutically acceptable salt thereof in an effective amount. The effective amount can be determined by reference to the doses and the like as mentioned above in connection with the inhibitor for renal injuries of the present invention.

### (Inhibitor for heme uptake into kidney tissues)

The present invention, in an embodiment, relates to an inhibitor for heme uptake into kidney tissues, comprising cilastatin or a pharmaceutically acceptable salt thereof as an active component.

Heme is a partial structure constituting myoglobin and can be released in the body by myoglobin degradation.

While heme binds to α₁M or Hx, the present inventors confirmed that α₁M and Hx bind to megalin, and cilastatin inhibits the binding between these and megalin. It is considered that this inhibitory effect leads to inhibition of renal injuries induced by rhabdomyolysis.

The inhibitor of the present invention contains cilastatin or a pharmaceutically acceptable salt thereof in an effective amount. The effective amount can be determined by reference to the doses and the like as mentioned above in connection with the inhibitor for renal injuries of the present invention.

### (Dosage form)

The form of the inhibitor of the present invention is not particularly limited, and the inhibitor of this invention can be in the form of, for example, a solid formulation such as powder, granule, capsule, tablet or chewable tablet, a liquid formulation such as solution or syrup, or an injectable, or a spray. A preferred form is an injectable.

### (Other components)

The inhibitor of the present invention may contain a pharmaceutically acceptable carrier when required for pharmaceutical purposes. Examples of such a carrier include excipient and solvent. Examples of additional components that may be contained in the inhibitor of this invention include binder, pH adjustor, disintegrant, chelator, solubilizer, suspending agent, emulsifier, isotonic agent, stabilizer, soothing agent, antiseptic, antioxidant, lubricant, corrigent, and colorant.

Examples of excipients include organic excipients, such as sugars like lactose, glucose and D-mannitol, starches, and celluloses like crystalline cellulose; and inorganic excipients, such as dicalcium phosphate, calcium carbonate and kaolin. Examples of solvents include purified water and normal saline. Examples of binders include pregelatinized starch, gelatin, gum Arabic, methylcellulose, carboxymethylcellulose, carboxymethylcellulose sodium, crystalline cellulose, D-mannitol, trehalose, hydroxypropylcellulose, hydroxypropylmethylcellulose, polyvinylpyrrolidone, and polyvinyl alcohol. Examples of pH adjustors include hydrochloric acid and sodium hydroxide. Examples of disintegrants include low-substituted hydroxypropylcellulose, chemically modified celluloses and starches, and alginic acid. Examples of chelators include calcium disodium edetate hydrate and calcium sodium edetate hydrate. Examples of solubilizers include polyethylene glycol, propylene glycol, trehalose, benzyl benzoate, ethanol, sodium carbonate, sodium citrate, sodium salicylate and sodium acetate. Examples of suspending agents or emulsifiers include sodium lauryl sulfate, gum Arabic, gelatin, lecithin, glyceryl monostearate, polyvinyl alcohol, polyvinylpyrrolidone, celluloses like carboxymethylcellulose sodium, polysorbates, and polyoxyethylene hydrogenated castor oil. Examples of isotonic agents include sodium chloride, potassium chloride, sugars, glycerin, and urea. Examples of stabilizers include polyethylene glycol, sodium dextran sulfate, and other amino acids. Examples of soothing agents include glucose, calcium gluconate and procaine hydrochloride. Examples of antiseptics include p-hydroxybenzoic esters, chlorobutanol, benzyl alcohol, phenethyl alcohol, dehydroacetic acid, and sorbic acid. Examples of antioxidants include sulfite and ascorbic acid.

In a preferred embodiment, the inhibitor of the present invention does not contain imipenem.

### (Method and use)

In another aspect, the inhibitor of the present invention is directed to use of cilastatin or a pharmaceutically acceptable salt thereof in the inhibition of renal injuries induced by rhabdomyolysis, the inhibition of myoglobin uptake into kidney tissues, or the inhibition of heme uptake into kidney tissues.

In another aspect, the inhibitor of the present invention is directed to a method for inhibiting renal injuries induced by rhabdomyolysis, inhibiting myoglobin uptake into kidney tissues, or heme uptake into kidney tissues, the method comprising administering an effective amount of cilastatin or a pharmaceutically acceptable salt thereof to a subject in need thereof.

The effective amount of cilastatin or a pharmaceutically acceptable salt thereof can be determined by reference to the doses and the like as mentioned above in connection with the inhibitor of the present invention.

In a preferred embodiment, the method and use of the present invention do not comprise administration of imipenem.

The subject in need of various inhibitions herein is preferably a mammal such as human, a domestic animal such as mouse, rat, rabbit, guinea pig, hamster, monkey, sheep, horse, cow, pig, donkey, dog or cat, or other laboratory animal, with a human being particularly preferred.

### (Numerical range)

For the sake of clarity, the numerical ranges defined herein by lower and upper limit values, like "from 1.0 to 2.0 g", include the lower and upper limit values.

### EXAMPLES

Hereunder, the present invention will be described by way of examples, but this invention is not limited to these examples.

### (Test Example 1) Inhibition of renal injuries induced by rhabdomyolysis by cilastatin

Serum potassium, which elevates when a renal function is reduced, was measured to verify whether the onset of renal injuries in rhabdomyolysis model mice is inhibited by cilastatin administration.

### Methodology

To C57BL/6 (male, aged 10-12 weeks), 5 mL/kg of a solution of 50% volume/volume glycerol (FUJIFILM Wako Pure Chemical Corporation)/PBS was prepared, and the solution was intramuscularly injected to the right hind limb, thereby constructing rhabdomyolysis nephropathy models. The administered animals were divided into 2 groups (6 mice per group), and intraperitoneally administered with a) 400 mg/kg (diluted in 100 µL of normal saline) of cilastatin (Sigma-Aldrich Inc.) or b) 100 µL of normal saline as a control.

Twenty-four hours after glycerol administration, blood was collected from the inferior vena cava, and centrifuged for 30 minutes at 800 g to separate and collect serum.

The serum was stored at -80°C until analysis and the measurement of serum potassium was conducted by Oriental Yeast Co., Ltd.

### Results

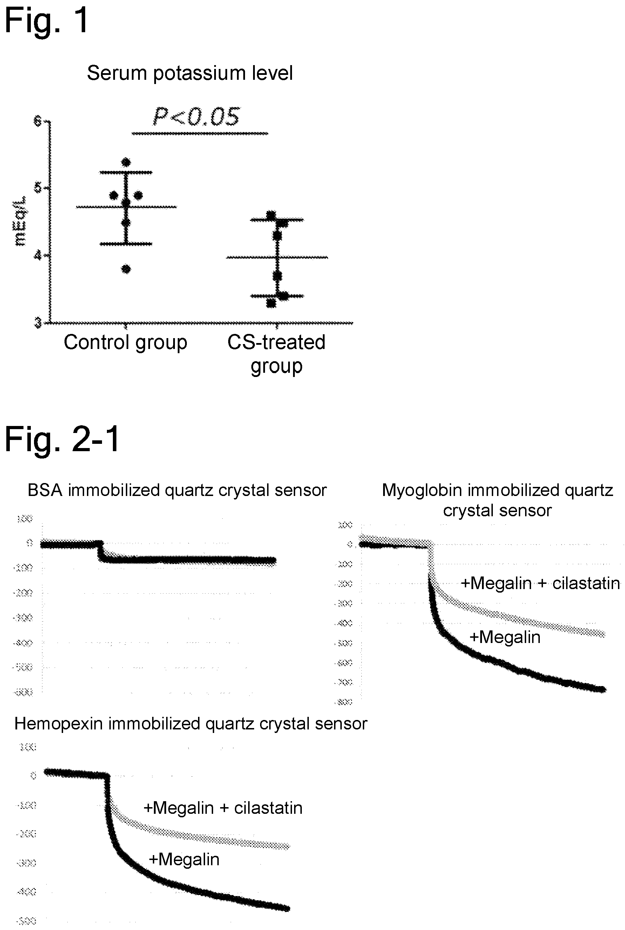

As a result of evaluating the serum potassium, the cilastatin (CS)-treated group had lower values than the control group (Fig. 1). It was found that that renal injuries induced by rhabdomyolysis can be inhibited by cilastatin administration.

The present inventors consider as follows. Myoglobin released by rhabdomyolysis is taken up into kidney tissues through megalin and causes renal injuries. Further, heme freed from myoglobin forms a complex by binding to **α**₁M or Hx, and the complex is taken up into kidney tissues through megalin and causes renal injuries. Then, cilastatin inhibits the binding between myoglobin or the complex and megalin, and inhibits renal injuries. In the subsequent test examples, these action mechanisms of cilastatin will be confirmed.

### (Test Example 2) Confirmation of the action mechanism (1)

### Direct binding property between each ligand and megalin, and binding inhibition by cilastatin

Binding specificity between megalin and each ligand (myoglobin, **α**₁M, and Hx) and the possible binding inhibition by cilastatin were verified using the quartz crystal microbalance (QCM) method by reference to the method described in PTL 2.

### Methodology-1

For the ligands to be verified, myoglobin (LifeSpan Biosciences Inc., Rat Hemoglobin Native Protein, LS-G11201) and Hx (Sino Biological Inc., Rat Hemopexin/HPX protein (His Tag 81025-R08H)) were used.

With the use of an immobilization kit for AFFINIX(R) (Initium Inc.) according to the recommended protocol for the kit, each ligand solution prepared in Buffer A as provided with the kit to a concentration of 0.1 mg/mL, or a solution of bovine serum albumin (BSA: CALBIOCHEM, Albumin, Bovine Serum, Fraction V, Fatty Acid-Free, Cat#126575) as a control was placed on the quartz crystal sensor mounted in a holder, and the ligand or albumin was immobilized on the quartz crystal sensor.

After each of the ligand- or BSA-immobilized quartz crystal sensor was mounted in the measurement instrument AFFINIX(R) Q8 (Initium Inc.) and the frequency was confirmed to be stable, 8 µL of a 0.1 mg/mL solution of megalin purified from Sprague-Dawley (SD) rat kidneys according to the method of Orlando *et al.* was injected and frequency was measured with time.

More specifically, the megalin solution was reacted with each of the ligand-immobilized quartz crystal sensors placed under the water surface with 200 µL of buffer, and the frequency was measured.

Additionally, 1 mg of cilastatin was injected into buffer in advance, each of the ligand-immobilized quartz crystal sensors was mounted in the measurement instrument, and after the frequency was confirmed to be stable, the megalin solution was injected and frequency was measured with time, whereby the antagonistic activity of cilastatin against the binding of each ligand to megalin was analyzed.

The same experiment was done on controls.

### Methodology-2

In line with Methodology-1, a quartz crystal sensor on which a rat-derived purified megalin or BSA was immobilized was prepared. This quartz crystal sensor was mounted in the measurement instrument, and a condition under which 1 mg/200 µL of cilastatin was injected into buffer in advance and a condition under which cilastatin was not injected were set. After the frequency was confirmed to be stable, an **α**₁M (LifeSpan Biosciences, Inc., Rat AMBP Protein (Recombinant 6His, N-terminus) (aa20-202), LS-G11794) solution was injected in an 8 µL chamber, and frequency was measured with time.

### Results-1

When megalin was added to the myoglobin-immobilized quartz crystal sensor and the Hx-immobilized quartz crystal sensor, signals of the quartz crystal significantly dropped and this reaction was inhibited by cilastatin (Fig. 2-1). It was found that myoglobin and Hx directly bind to megalin, and cilastatin inhibits such a binding.

### Results-2

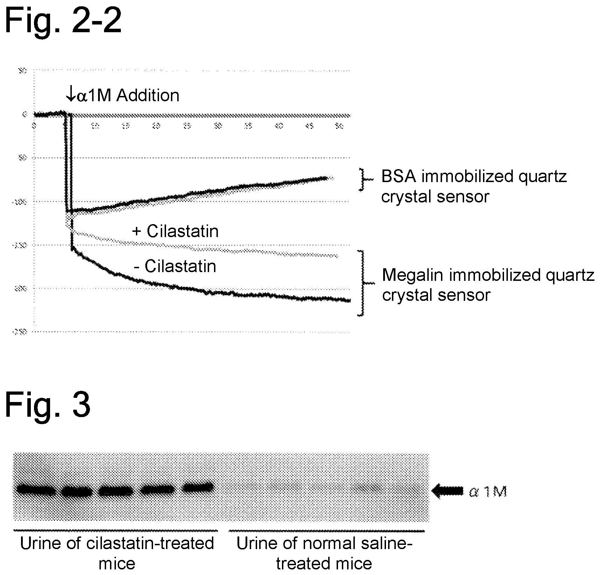

When α₁M was added to the megalin-immobilized quartz crystal sensor, signals of the quartz crystal significantly dropped and this reaction was inhibited by cilastatin (Fig. 2-2). It was found that α₁M directly binds to megalin as myoglobin and Hx do, and cilastatin inhibits such a binding.

### (Test Example 3) Confirmation of the action mechanism (2)

### Relation of the uptake of each ligand into kidney tissues and megalin and cilastatin

This test example verified that each of the ligands (α₁M and Hx) was taken up into kidney tissues through megalin, and cilastatin inhibits such an uptake.

### Methodology

Cilastatin was administered to mice and changes in concentrations of each ligand in urine were observed.

Five mice (male, aged 12 weeks C57BL/6) were intraperitoneally administered with 400 mg/kg (diluted in 100 µL of normal saline, cilastatin-treated group) of cilastatin sodium (Sigma-Aldrich Japan K.K., C5743), and then put in a metabolic cage for 3 hours to collect urine, and the urines of 5 mice were combined. Additionally, 100 µL of normal saline was administered to another group of 5 mice (control group), and the same experiment as above was conducted.

Proteins were isolated from the obtained urine samples by electrophoresis (SDS-polyacrylamide gel), reductively alkylated (using dithiothreitol and iodoacetamide), digested with trypsin, and the obtained peptides were extracted to obtain a solid.

To the obtained solid, 15 µL of 0.3% formic acid (FA) was added, shaken for 5 minutes, sonicated for 1 minute, and shaken again for 5 minutes. The resultant mixture was spun down, applied to a membrane filter having a pore diameter of 0.45 µm (ULtra free-MC, HV, 0.45 µm, Millipore), centrifuged at 10,000 rpm for 5 minutes, and the sample passed through the filter was subjected to LCMS analysis.

### Analysis by LCMS

### (LC System)

### Eksigent (R) Exspert nano LC 400, Exsigent TM Exspert cHiPLC (R)

### (Column)

Trap column: Nano cHiPLC Trap column 200 µm × 0.5 mm ChromXP C18-CL 3 µm 120 Å (804-00006, SCIEX)
Analytical column: Nano cHiPLC column 75 µm × 15 cm ChromXP C18-CL 3 µm 120 Å (804-00001, SCIEX)
Column temperature 30°C

### (Sample introduction)

Injection volume: 3 µL

After holding the sample in a trap column, the gradient of mobile phase was adjusted as described later, and the sample was introduced to an analytical column.

### (Mobile phase)

A Solvent: Aqueous solution of 0.1% trifluoroacetic acid
B Solvent: Solution of 0.1% trifluoroacetic acid in acetonitrile
Flow rate: Total 300 nl/min

Gradient condition: When the sample was introduced to the analytical column equilibrated with 98% of A solvent and 2% of B solvent was 0 minute; the ratio of B solvent was linearly increased to 32% in 30 minutes; and the substance to be measured was separated and eluted. Subsequently, the ratio of B solvent was increased to 90% in 5 minutes; then the ratio of B solvent was retained at 90% for 5 minutes; the column was washed; the ratio was brought back to 2% in 0.1 minutes; and the ratio of B solvent was maintained at 2% as in the initial state until 60 minutes, thereby equilibrating the column.

### (Mass spectrometer)

AB Sciex Triple TOF 5600+ was used in positive ion mode.

### (TOF-MS Condition)

Mass range: 400-1250 Da

### (Data analysis)

LCMS data were checked against the sequence database, and the presence of a plural proteins was confirmed. Moreover, the proteins confirmed in both of the cilastatin-treated group and control group were calculated for an SpC/L ratio (spectral abundance factor: SAF), which is equivalent to the peak intensity ratio of the cilastatin-treated group/control group, and those having an SpC/L ratio of 2.0 or more were decided to be proteins more abundant in urine of the cilastatin-treated group and listed as the proteins as such.

The analysis method described in Ohara et al., 2018, PLoS One, vol. 13, 1, e0204160 was referred to. The analysis method is shown below.

AnaLyst (R) TF1.6 (AB SCIEX) was used as an LCMS/MS data acquisition and analysis software, and the obtained raw data were converted to a peak list file (mgf file format), whereby MS/MS Ion Search (MIS search) was conducted using Matrix Science Mascot daemon, Mascot server 2.2.1. The sequence data base used was Uniprot, Swiss-Prot. Significance threshold was set to 0.05 (false discovery rate of less than 5%), and the proteins having 2 or more peptides exceeding the identification standard were listed as identified proteins.

Raw data were acquired 2 times per sample at LCMS to cover the issue of analysis reproducibility at LCMS, and the peak list files obtained from the 2 runs were combined and analyzed using Mascot (Ohara et al., 2018, PLoS One, vol. 13, 1, e0204160).

To the proteins identified in common in 2 groups when compared, the spectral count method was used, which is a label-free comparative, semiquantitative analysis method utilizing a principle that a large number of peptide fragments will be identified in those expressed at high levels, resulting in the increase in the number of MS/MS. In other words, the proteins identified in both of the cilastatin-treated group and control group were calculated for an SpC/L ratio (spectral abundance factor: SAF) of the cilastatin-treated group/control group, and the proteins having an SpC/L ratio of 2.0 or more were listed as the proteins abundant in urine of the cilastatin-treated group.

### (Urine analysis by WB using anti-α₁M antibody)

For the urines of the cilastatin-treated group and control group obtained in above, SDS-polyacrylamide electrophoresis under reduction condition was conducted using a 4 to 15% gradient gel (Bio-Rad Laboratories, Inc.), and developed fragments were transferred to a polyvinylidene fluoride (PVDF) membrane. The membrane was blocked using a 5% nonfat milk-containing buffer solution, and then reacted to 1 µg/mL of an anti-α₁M antibody (LifeSpan Biosciences Inc.) as the primary antibody at room temperature for 2 hours. The membrane was reacted to 0.25 µg/mL of Polyclonal Goat Anti-Rabbit Immunoglobulin (Dako Japan Inc.) labelled with horseradish peroxidase (HRP) as the secondary antibody at room temperature for 1 hour. Subsequently, bands were detected using a western blot chemiluminescent substrate (SuperSignalTM West Femto Maximum Sensitivity Substrate, Thermo Scientific (R)).

### Results

Of the proteins identified in both of the cilastatin-treated group and control group, 63 kinds having an SpC/L ratio of the cilastatin-treated group/control group of 2.0 or more were identified as the proteins with higher urinary concentrations in the cilastatin-treated group as compared to the control group. It is considered that the changes in urinary excretion of these proteins are affected by the megalin antagonistic activity by cilastatin. Further, in these, **α**₁M and Hx, which are reported as the heme binders, were contained. As a reference, Fig. 3 shows western blotting images of urinary **α**₁M in the cilastatin-treated group and control group.

Therefore, while it is reported that **α**₁M is the ligand of megalin (Christensen et al., 2002, MOLECULAR CELL BIOLOGY, vol. 3, p.258-268), the present study showed that Hx, not only **α**₁M, is also the possible ligand of megalin. Additionally, it was confirmed that cilastatin inhibits the uptake of these into kidney tissues.

The proteins which did not change in the urinary concentrations in the cilastatin-treated group and control group contained those reported as megalin ligands such as albumin, transthyretin, clusterin, vitamin D-binding protein and the like. The present study showed that the binding, even it is a ligand of megalin, may not be antagonized by cilastatin.

Of the proteins having an SpC/L ratio of 2.0 or more and the proteins having an SpC/L ratio of less than 2.0 in the present test, the measured numerical values of examples of those already reported as ligands of megalin, or those found as ligands of megalin by the study of the present inventors are shown below.

**[Table 1]**

| Proteins having SpC/L ratio of 2.0 or more | | |
|---|---|---|
| Protein name | | SpC/L ratio |
| | α₁M | 3.67 |
| | Hx | 2.33 |
| | Transcobalamin-2 | 2.33 |

**[Table 2]**

| Proteins having SpC/L ratio of less than 2.0 | | |
|---|---|---|
| Protein name | | SpC/L ratio |
| | Albumin | 1.10 |
| | Transthyretin | 1.00 |
| | Clusterin | 0.73 |
| | Vitamin D-binding protein | 0.80 |

### (Test Example 4) Confirmation of the action mechanism (3)

### Megalin-dependent heme-derived iron uptake into kidney tissues

Megalin dependency of the heme-derived iron uptake into kidney tissues was verified using rhabdomyolysis models constructed from kidney-specific megalin-knockout (KO) mice.

### Methodology

Kidney-specific megalin KO mice (*Ndrg1-CreERT2, megalin lox*/*lox)* and control mice *(megalin (lox*/*lox); Cre(-))* as the control (both male, aged 12 weeks) were used. A solution of 5 mL/kg of 50% volume/volume glycerol (FUJIFILM Wako Pure Chemical Corporation)/PBS was prepared, and the solution was intramuscularly injected to the right hind limb, thereby constructing rhabdomyolysis nephropathy models.

Twenty-four hours after glycerol solution dosing, a portion containing a kidney pedicle was sliced to 3 mm thickness from the right kidney and fixed with 4% paraformaldehyde phosphate buffer. The fixed sample was thinly sliced at a thickness of 4 µm using a microtome (REM-710; Yamato Kohki Industrial Co., Ltd.), and the obtained thin slices were subjected to Berlin blue staining (*) and microscopically observed.
* Trivalent iron forms a complex with a protein and is present in the body. Acid decomposition of the complex facilitates the subsequent reaction to potassium ferrocyanide. Trivalent iron ion (Fe³⁺) binds to potassium ferrocyanide, thereby forming indigo blue iron ferrocyanide (Berlin blue).

### Results

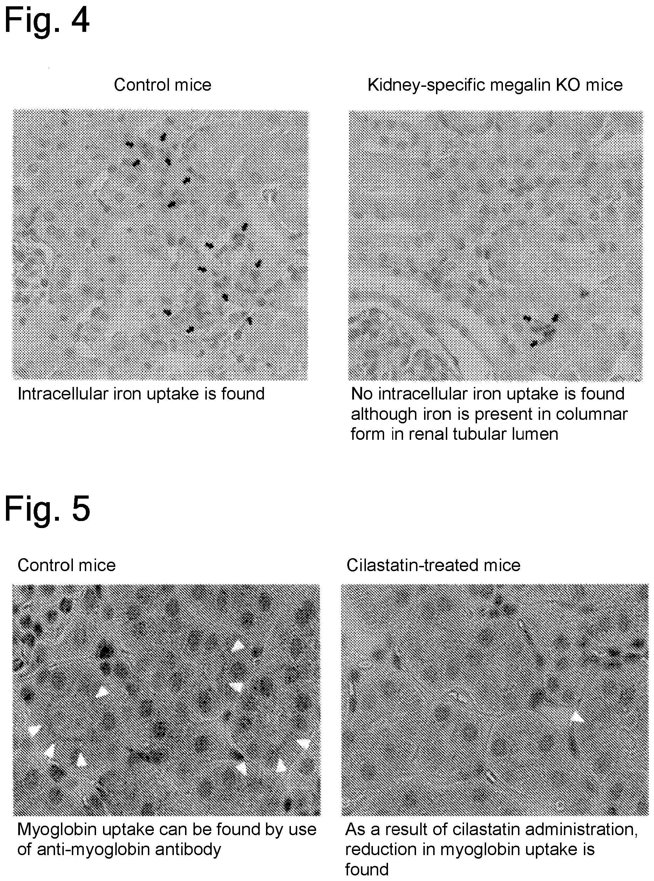

The iron uptake into kidneys in the rhabdomyolysis nephropathy model mice was mostly observed in renal proximal tubular segments 1 and 2 in the control mice, whereas no uptake was found in the megalin KO mice but iron was only present in renal tubular lumen in a columnar shape (Fig. 4). Since the iron uptake into kidneys is dependent on megalin, it was found that the uptake into kidneys of myoglobin or heme caused by rhabdomyolysis is dependent on megalin.

### (Test Example 5) Confirmation of the action mechanism (4)

### Inhibition of myoglobin uptake into kidney tissues by cilastatin

The inhibition of myoglobin uptake into kidney tissues by cilastatin administration was verified using rhabdomyolysis model mice.

### Methodology

In line with Test Example 1, 50% glycerol was administered to C57BL/6 (male, aged 10-12 weeks), thereby constructing rhabdomyolysis model mice.

Twenty-four hours after glycerol dosing, a portion containing a kidney pedicle was sliced to 3 mm thickness from the right kidney and fixed with 4% paraformaldehyde phosphate buffer. The fixed sample was thinly sliced at a thickness of 4 µm using a microtome (REM-710; Yamato Kohki Industrial Co., Ltd.), and the obtained thin slices were immunostained (500-fold diluted) with an anti-myoglobin antibody (Abcam ab77232) and microscopically observed.

### Results

It was confirmed that the amounts of myoglobin taken up into kidney tissues of the rhabdomyolysis model mice reduced by cilastatin administration (Fig. 5). Therefore, it was found that the myoglobin uptake into kidneys can be inhibited by cilastatin.

### (Test Example 6) Confirmation of the action mechanism (5)

### Megalin dependency of injury marker KIM-1 in kidney tissues

Megalin dependency of the expression of injury marker KIM-1 in kidney tissues was verified using rhabdomyolysis nephropathy models constructed from kidney-specific megalin KO mice.

### Methodology

Kidney-specific megalin KO mice (*Ndrg1-CreERT2, megalin lox*/*lox)* and control mice thereof *(megalin (lox*/*lox); Cre(-)*) as the control (both male, aged 12 weeks) were used. A solution of 5 mL/kg of 50% volume/volume glycerol (FUJIFILM Wako Pure Chemical Corporation)/PBS was prepared, and the solution was intramuscularly injected to the right hind limb, thereby constructing rhabdomyolysis nephropathy models.

Twenty-four hours after glycerol solution dosing, a portion containing a kidney pedicle was sliced to 3 mm thickness from the right kidney and fixed with 4% paraformaldehyde phosphate buffer. The fixed sample was thinly sliced at a thickness of 4 µm using a microtome (REM-710; Yamato Kohki Industrial Co., Ltd.), and the obtained thin slices were immunostained (700-fold diluted) with an anti-KIM-1 antibody (goat antimouse KIM-1 antibody (AF1817; R&D Systems) and microscopically observed.

### Results

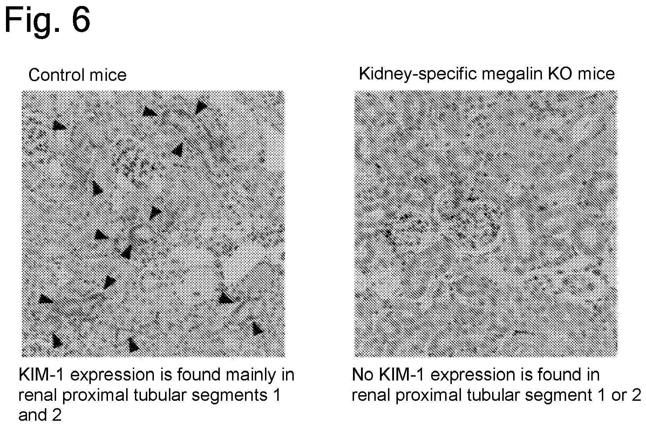

It was confirmed that the expression of KIM-1 in kidney tissues in the rhabdomyolysis nephropathy model mice was observed mainly in renal proximal tubular segments 1 and 2 in the control mice, but not in the megalin KO mice (Fig. 6). Since the expression of KIM-1 in kidney tissues is dependent on megalin, it was found that occurrence of injuries in kidney tissues is dependent on megalin.

### INDUSTRIAL APPLICABILITY

The present invention is capable of providing an inhibitor for renal injuries induced by rhabdomyolysis, or an inhibitor for myoglobin or heme uptake into kidney tissues.

## Claims

1. Cilastatin or a pharmaceutically acceptable salt thereof for use in inhibiting renal injuries induced by rhabdomyolysis.

2. Cilastatin or a pharmaceutically acceptable salt thereof for use according to claim 1, which is in an injectable form.

3. Cilastatin or a pharmaceutically acceptable salt thereof for use according to claim 1, wherein the renal injuries induced by rhabdomyolysis are renal proximal tubular epithelial cell injuries.

## Patentansprüche

1. Cilastatin oder ein pharmazeutisch verträgliches Salz davon zur Verwendung bei der Hemmung von durch Rhabdomyolyse induzierten Nierenverletzungen.

2. Cilastatin oder ein pharmazeutisch verträgliches Salz davon zur Verwendung nach Anspruch 1, welches in einer injizierbaren Form vorliegt.

3. Cilastatin oder ein pharmazeutisch verträgliches Salz davon zur Verwendung nach Anspruch 1, wobei die durch Rhabdomyolyse induzierten Nierenverletzungen Verletzungen der proximalen Tubulusepithelzellen der Niere sind.

## Revendications

1. Cilastatine ou un sel pharmaceutiquement acceptable de celle-ci pour une utilisation dans l'inhibition des lésions rénales induites par la rhabdomyolyse.

2. Cilastatine ou un sel pharmaceutiquement acceptable de celle-ci pour une utilisation selon la revendication 1, qui est sous forme injectable.

3. Cilastatine ou un sel pharmaceutiquement acceptable de celle-ci pour une utilisation selon la revendication 1, dans laquelle les lésions rénales induites par la rhabdomyolyse sont des lésions des cellules épithéliales tubulaires proximales rénales.
